# EUROPEAN PATENT APPLICATION

(11) **EP 4 502 175 A1**
(43) Date of publication of application: **05.02.2025**
(21) Application number: 24186476.8
(22) Date of filing: 04.07.2024
(51) Int. Cl.: C12Q 1/66, G01N 33/50, C12Q 1/18

(54) **CYTOTOXICITY ASSAY FOR DETECTING CELLULAR DAMAGE**

(30) Priority: 03.08.2023 DE 102023120682
(71) Applicant: Leibniz-Institut für Naturstoff-Forschung und Infektionsbiologie - Hans-Knöll-Institut -, 07745 Jena (DE)
(72) Inventor: VIJ, Raghav, 07743 Jena (DE); TESFAMARIAM, Millen, 07747 Jena (DE); TRÜMPER, Verena, 07751 Jena (DE); BRUNKE, Sascha, 07749 Jena (DE); HUBE, Bernhard, 07749 Jena (DE)
(74) Representative: Krauss, Jan

(57) **Abstract**

The present invention relates to an *in vitro* method for detecting and/or determining an agent causing cellular damage in a eukaryotic, in particular, mammalian cell, comprising providing a eukaryotic cell that stably expresses an intracellular luciferase enzyme, contacting the cell with an agent suspected to cause cellular damage, and detecting luciferase activity wherein a change of the luciferase activity as detected when compared to a non-damaged genetically modified eukaryotic cell is indicative for an agent causing cellular damage in the eukaryotic cell. The present invention further relates to methods for detecting and/or determining cytoprotection in a eukaryotic cell caused by an agent, methods for screening for an agent causing cellular cytoprotection in a eukaryotic cell, methods for testing the pharmacological safety of an agent based on the method as above, a diagnostic kit and respective uses thereof in the methods of the invention.

## Description

The present invention relates to an *in vitro* method for detecting and/or determining an agent causing cellular damage in a eukaryotic, in particular, mammalian cell, comprising providing a eukaryotic cell that stably expresses an intracellular luciferase enzyme, contacting the cell with an agent suspected to cause cellular damage, and detecting luciferase activity wherein a change of the luciferase activity as detected when compared to a non-damaged genetically modified eukaryotic cell is indicative for an agent causing cellular damage in the eukaryotic cell. The present invention further relates to methods for detecting and/or determining cytoprotection in a eukaryotic cell caused by an agent, methods for screening for an agent causing cellular cytoprotection in a eukaryotic cell, methods for testing the pharmacological safety of an agent based on the method as above, a diagnostic kit and respective uses thereof in the methods of the invention.

### Background of the invention

There are many reasons microorganisms damage host cells, e.g. to acquire nutrients for growth, to invade and colonize different niches, and to protect themselves against immune cells.

Infectious diseases affect billions of people every year, and cause millions of deaths each year. The fight against infections requires both, research into the biology of the causative microorganisms and the development of new treatments. For fungal infections, for example, only four classes of antifungals available to treat a wide range of different infections (Rodrigues and Nosanchuk, 2020). *Candida* species are among the most common fungal pathogens and cause tens of millions of mucosal and nearly 700,000 invasive infections annually, with an unacceptable high morbidity and mortality (Brown et al., 2012). *Candida* sp. usually resides within the human hosts as harmless commensals. From there, prolonged use of antibiotics or a weakened immune system can trigger mucosal or systemic candidiasis. *C*. *albicans,* the most prevalent and well-studied member of the clade, can cause deadly infections, and is armed with virulence traits that help it survive and persist in the host. Among others, *C*. *albicans* adheres to host cells, invades and damages them, all while evading our immune responses (Brunke et al., 2016).

To investigate virulence traits, readouts for host cell cytotoxicity (*i*.*e*., cell damage) have been indispensable. Methods like detection of LDH release (Allert et al., 2018; Moyes et al., 2016), release of radiolabeled chromium (⁵¹Cr) (Sanchez et al., 2004; Yamamura et al., 1976), and fluorescence microscopy of propidium iodide (PI)-stained nuclei of dying cells (Case et al., 2021), have all been used in the past with great success, for example in the discovery of the fungal peptide toxin candidalysin (Naglik et al., 2019). These assays come with specific drawbacks that hinder their application in high-throughput-screens: LDH cytotoxicity kits are comparatively expensive and inhibited by medium components like phenol red and pyruvate; quantifying fluorescence of nuclear cell-death dyes like PI is confounded by rapidly proliferating microbes in *in vitro* models that block the light signal; and both techniques are confounded by microbial LDH and nuclear staining. While ⁵¹Cr is highly sensitive and specific, it is also expensive, and comes with the dangers and inconvenience of working with radioactivity. New high-throughput and inexpensive host cell cytotoxicity reporters can expedite discovery of virulence factors and generally simplify cell damage assays.

In 1880s the French physiologist DuBois described the molecular basis of luminesce in fireflies, marine animals, and other organisms (Dubois, 1885). The reaction of an enzyme called luciferase with its substrate luciferin leads to a emission of visible light (Fraga, 2008). With advancements in molecular biology, scientists engineered ways to express luciferase in cells, and ever since the inventors have found a broad range of applications for luciferase-based assays to study protein expression, and for high-throughput drug discovery screening (Baki et al., 2007; Cox et al., 2021; Matta et al., 2018).

Tsuji S, et al. (in: A Cytoplasmic Form of Gaussia luciferase Provides a Highly Sensitive Test for Cytotoxicity. PLoS One. 2016 May 26;11(5):e0156202. doi: 10.1371/journal.pone.0156202. PMID: 27228203) disclose a luminometric cytotoxicity test using a stable form of luciferase. Specifically, the authors converted Gaussia luciferase (G-Luc) from an actively secreted form to a cytoplasmic form by adding an ER-retention signal composed of the four amino acids KDEL. The bioluminescent signal was >30-fold higher in transgenic HepG2 human hepatoblastoma cells expressing G-Luc+KDEL than in cells expressing wild-type G-Luc. Moreover, G-Luc+KDEL secreted from damaged cells was stable in culture medium after 24 hr at 37°C. Their findings allegedly demonstrate that strong and stable luminescence of G-Luc+KDEL in human hepatocyte-like cells, which have high levels of metabolic activity, make it suitable for use in a high-throughput screening system for monitoring time-dependent cytotoxicity in a limited number of cells.

WO 2018/053542A1 discloses so-called Matador assays, for determining the cytotoxic effect of a given test compound (such as chemotherapy agents, antibodies, biologicals, or cytotoxic cells e.g. T cell). The method includes expression of a reporter in target cells in a manner so that it is preferentially retained within the healthy cells but is either released from dead and dying cells or whose activity can be preferentially measured in dead and dying cells. In one embodiment, the inventive method measures the activity of the reporter that has been released from the dead and dying cells. In some embodiments, the reporters are non-secreted forms of luciferases. WO 2018/053542A1 does not disclose the use of the assay in case of infection.

Infection biologists strive to understand the microbial virulence programs that cause damage to host cells and discover drugs that mitigate this damage. This requires easy, reliable, and accurate readouts for cytotoxicity or cell damage in *in vitro* assays. Many popular high-throughput measure of cytotoxicity (reviewed in (Niles et al., 2008; Riss et al., 2019)) rely on the enzyme activity of cellular components released when host cells die, like lactate dehydrogenase (LDH), adenylate kinase (AK) or glyceraldehyde-3-phosphate dehydrogenase (GAPDH). However, these enzymes can also be found in many bacteria, fungi and parasites, confounding cytotoxicity measurements in infection models. The enzyme assays also generally require multiple wash steps, and often expensive buffers and substrates.

It is therefore an object of the present invention to provide an improved easy to use, cheap and reliable method for detecting and/or determining cellular damage, cytotoxicity and even cytoprotection in a cell that reflects the conditions *in vivo* and can be used to properly investigate the effect of, for example, cytotoxic agents and infections. Other objects and advantages will become apparent to the person of skill when further studying the present disclosure.

In a first aspect of the present invention, the present invention solves the above object by providing an *in vitro* method for detecting and/or determining an agent causing cellular damage in a eukaryotic, in particular, mammalian cell, comprising the steps of a) providing a eukaryotic, in particular, mammalian cell that is genetically modified to stably express an intracellular luciferase enzyme, b) contacting the cell of a) with at least one agent suspected to cause cellular damage, c) suitably culturing the cell of b) in a culture medium, and d) detecting luciferase activity in the culture medium, wherein a change of the luciferase activity as detected in the culture medium when compared to a genetically modified mammalian cell that was not contacted with the at least one agent is indicative for an agent causing cellular damage in the mammalian cell. Preferred is the method according to the present invention, wherein the cellular damage is caused by agent related to senescence, infection, environmental factors, cytotoxicity, and/or cancer, radiation or physical damage, a pharmaceutically active compound, a drug, in particular a small molecule drug, an antibiotic, an antibody, a peptide, and/or an immune cell.

In a second aspect of the present invention, the present invention solves the above object by providing an *in vitro* method for detecting and/or determining a cytoprotective agent for a eukaryotic, in particular mammalian cell, comprising the steps of a) providing a eukaryotic, in particular, mammalian cell that is genetically modified to stably express an intracellular luciferase enzyme, b) contacting the cell of a) with at least one first agent causing cellular damage, b) suitably culturing the cell of a) in a culture medium, c) contacting the agent to be tested with the cell as cultured in b), and optionally suitably culturing the cells in a culture medium d) detecting luciferase activity in the culture medium, and wherein a change of the luciferase activity as detected in the culture medium when compared to a genetically modified mammalian cell cultured without the agent to be tested is indicative for cytoprotection in the mammalian cell caused by the agent. Preferred is the method according to the present invention, wherein the agent causing cellular damage or cytoprotection is selected from agents related to senescence, infection, environmental factors, cytotoxicity, cancer, a pharmaceutically active compound, a drug, in particular a small molecule drug, an antibiotic, an antibody, a peptide, and/or an immune cell.

Preferred is the method according to the present invention, wherein the cell is infected with an infectious agent, such as, for example a bacterium, a fungus, or a virus, such as, for example, *Candida, Yersinia,* Zika, HIV, Coronavirus, RSV, or West Nile Virus.

Further preferred is a method according to the present invention, further comprising the step of concluding on the pharmacological safety of the agent based on the detecting.

Preferred is a method according to the present invention, further comprising at least one additional step of determining the number of cells in the culture medium based on determining the luciferase activity of the genetically modified cells as cultured, preferably in a lysate.

In a third aspect of the present invention, the present invention solves the above object by providing a diagnostic kit comprising materials for performing the method according to the present invention, in particular luciferase substrate, a genetically modified mammalian cell line stably expressing Nluc, buffers, triton, positive controls, culture medium, optionally an agent to be tested, and/or instructions for use.

In a fourth aspect of the present invention, the present invention solves the above object by providing the use of the diagnostic kit according to the present invention for detecting and/or determining cellular damage in a mammalian cell, for detecting and/or determining a cytoprotective agent in a damaged cell, for screening for an agent causing cytoprotection in a cell, or for determining the pharmacological safety of an agent according to the present invention.

As mentioned above, the present invention provides an *in vitro* method for detecting and/or determining an agent causing cellular damage in a eukaryotic, in particular, mammalian cell, comprising the steps of a) providing a eukaryotic, in particular, mammalian cell that is genetically modified to stably express an intracellular luciferase enzyme, b) contacting the cell of a) with at least one agent suspected to cause cellular damage, c) suitably culturing the cell of b) in a culture medium, and d) detecting luciferase activity in the culture medium, wherein a change of the luciferase activity as detected in the culture medium when compared to a genetically modified mammalian cell that was not contacted with the at least one agent is indicative for an agent causing cellular damage in the mammalian cell.

The present invention also provides an *in vitro* method for detecting and/or determining a cytoprotective agent for a eukaryotic, in particular mammalian cell, comprising the steps of a) providing a eukaryotic, in particular, mammalian cell that is genetically modified to stably express an intracellular luciferase enzyme, b) contacting the cell of a) with at least one first agent causing cellular damage, b) suitably culturing the cell of a) in a culture medium, c) contacting the agent to be tested with the cell as cultured in b), and optionally suitably culturing the cells in a culture medium d) detecting luciferase activity in the culture medium, and wherein a change of the luciferase activity as detected in the culture medium when compared to a genetically modified mammalian cell cultured without the agent to be tested is indicative for cytoprotection in the mammalian cell caused by the agent.

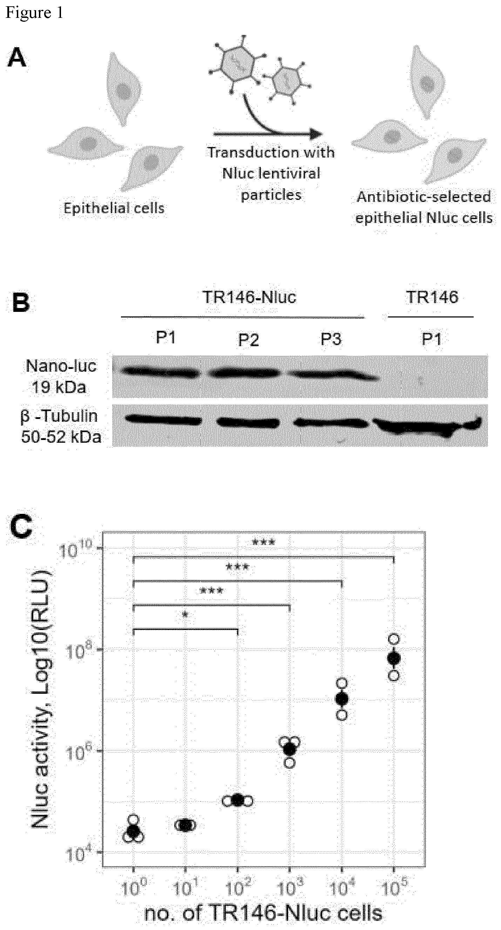

In one embodiment, with the present invention, a high-throughput and inexpensive Nano-luciferase (Nanoluc or Nluc, Promega) reporter system for detecting cytotoxicity is provided. The inventors further studied the use and reliability thereof in *Candida albicans* infection models as a study of potential applications. In the context of the present invention, the inventors thus designed and validated an epithelial damage reporter based on Nano-luciferase (Nluc) (England et al., 2016). The inventors transduced oral, vaginal, and gut human epithelial cell lines (TR146, A431, and C2BBe1) using a lentiviral vector to constitutively and stably express Nluc in the cytoplasm (Figure 1A). Since damaged (epithelial) cells "leak" proteins like Nluc and LDH into the extracellular space, the inventors approximated host cell damage by assaying Nluc and LDH enzymatic activity in the supernatant. The inventors showed that with the Nluc assay epithelial damage over the time course of *C*. *albicans* infections can be followed up.

Advantageously, the reporter construct of the present invention can be readily used for a very large number of eukaryotic cells. Preferably, the eukaryotic cell is selected from the group consisting of insect cells, fungal cells, e.g., yeast, and mammalian cell selected from the group consisting of epithelial cells, vaginal cells, intestinal cells, lung cells, immortalized cells, cancer cells, and cell lines thereof.

The finding that the luciferase reporter assay presented in the present invention is both more sensitive and specific than the tests commonly used for the investigation of cell damage, such as the LDH test, is quite surprising, since it was not obvious that this positive effect would be achieved by using, for example, the principle of the Matador test (see above). In addition, the presented assay has other advantages over the LDH assay, such as a relative insensitivity to different pH values.

In the context of the present invention, the term "cellular damage" in a eukaryotic, such as mammalian, cell shall include any deviation in a cell that ultimately - directly or indirectly-causes enzyme release through the cellular membrane. Release of intracellular enzymes to the extracellular space is a marker of cell damage in various diseases or other disadvantageous states of a cell (see, for example, Kristensen SR. Mechanisms of cell damage and enzyme release. Dan Med Bull. 1994 Sep;41(4):423-33. PMID: 7813251).

Preferred is the method according to the present invention, wherein the agent causing cellular damage or cytoprotection is selected from agents related to senescence, infection, environmental factors, cytotoxicity, and/or cancer, radiation or physical damage, a pharmaceutically active compound, a drug, in particular a small molecule drug, an antibiotic, an antibody, a peptide, and an immune cell. All these diseases or conditions as well as compounds may lead to cause enzyme release through the cellular membrane.

In the context of the present invention, any eukaryotic cell can be used that can be genetically modified, for example transfected recombinantly, and then stably expresses a luciferase using a suitable constitutive or inducible promoter. Particularly preferred is lentiviral transfection with Nluc or NanoLuc luciferase, which offers several advantages over established systems, including enhanced stability, smaller size, and >150-fold increase in luminescence. In addition, the substrate for NLuc displays enhanced stability and lower background activity (England CG, Ehlerding EB, Cai W. NanoLuc: A Small Luciferase Is Brightening Up the Field of Bioluminescence. Bioconjug Chem. 2016 May 18;27(5):1175-1187. doi: 10.1021/acs.bioconjchem.6b00112. Epub 2016 Apr 19. PMID: 27045664; PMCID: PMC4871753). In particular the smaller size is advantageous when analyzing enzyme (NLuc) release from a damaged cell as herein. Other luciferases that could also be used are, for example, non-modified natural luciferases, such as Rluc or Flue and/or Gluc.

In the context of the present invention, eukaryotic cells can be used that show enzyme release as a response to cellular damage. Many of the cells are known to the person of skill and are disclosed herein. Preferred is the method according to the present invention, wherein a mammalian cell is used selected from the group consisting of epithelial cells, vaginal cells, intestinal cells, lung cells, immortalized cells, cancer cells, and cell lines thereof.

In the context of the present invention, the intracellular luciferase enzyme is stably expressed, e.g. where the Nluc expression construct is integrated into the host genome, either constitutively or inducible, i.e., with or without induction. Systems and promotors etc. for such expression strategies are described in the literature and known to the person of skill.

The mammalian cells as used in the context of the present invention are suitably cultured, i.e. in a culture medium at the right temperature(s) and conditions. Suitable media are described in the literature and known to the person of skill, such as DMEM and Ham's F 12 medium, DMEM + 10 µg/ml human holo transferrin, or RPMI with 2 g/l glucose. The culture may be incubated at about 37°C, and at ambient conditions or in a suitable CO₂ adjusted incubator.

In the next step, the luciferase activity in the culture medium is detected. Such detecting steps are known to the person of skill as well, and may comprise centrifuging down of the cell debris, collecting a suitable amount of supernatant, diluting the supernatant with a suitable buffer, adding luciferase substrate (e.g. coelenterazine), and, preferably immediately, measuring the luminescence as generated in a suitable device, such as a luminometer. Although not preferred, detection of luciferase may also be done using suitable antibody-tests, where the presence and/or amount of the Nluc enzyme is detected in the medium.

Preferred is the method according to the present invention, wherein an increase of the luciferase activity as detected in the culture medium is indicative for an agent causing cellular damage, since an increase of enzyme release can be expected with cellular damage. Nevertheless, there may be situations, where a decrease of the luciferase activity as detected in the culture medium is indicative for cellular damage, for example if cellular export is disturbed because of the damage.

Preferred is the method according to the present invention wherein the cell is infected with an infectious agent, such as, for example a bacterium, a fungus, or a virus. Examples for bacteria are for example, intracellular bacteria such as *Yersinia.* Examples for fungal infections are, for example, *Candida* (see also further below), and examples for viruses are Zika, HIV, coronavirus, RSV, HPV or West Nile Virus. Details regarding the method are as described above and herein with respect to the cellular damage detection.

Further preferred is the method according to the present invention, wherein an increase of the luciferase activity as detected in the culture medium is indicative for an agent causing cellular damage, since an increase of enzyme release can be expected with cellular damage. In contrast, a decrease of the luciferase activity as detected in the culture medium is indicative for cytoprotection, for example if membrane integrity is improved or restored, thus leading to less enzyme release, or if the infection or damage process due to an agent is inhibited or reversed.

Details regarding this method are as described above and herein with respect to the cellular damage detection.

In the context of the present invention, the luciferase activity in the culture medium is detected. Such detecting steps are known to the person of skill as well, and may comprise centrifuging down of the cell debris, collecting a suitable amount of supernatant, diluting the supernatant with a suitable buffer, adding luciferase substrate (e.g. coelenterazine), and, preferably immediately, measuring the luminescence as generated in a suitable device, such as a luminometer. Although not preferred, detection of luciferase may also be done using suitable antibody-tests, where the presence and/or amount of the Nluc enzyme is detected in the medium.

Further preferred is the method according to the present invention, wherein an increase of the luciferase activity as detected in the culture medium is indicative for an agent causing cellular damage, since an increase of enzyme release can be expected with cellular damage. In contrast, a decrease of the luciferase activity as detected in the culture medium is indicative for cytoprotection, for example if membrane integrity is improved or restored, thus leading to less enzyme release.

In another important aspect of the present invention, the object of the present invention is solved by providing a method for producing a pharmaceutical composition, comprising performing a method according to the present invention, and admixing the agent either causing cytoprotection (e.g. anti-infective) as identified with at least one pharmaceutically carrier.

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. Pharmaceutically acceptable diluents include saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The pharmaceutical compositions according to the invention may be in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray.

Preferred is a method according to the present invention as above, wherein a decrease of the luciferase activity as detected in the culture medium is indicative for an agent causing cytoprotecti on.

Another important aspect of the present invention then relates to an *in vitro* method as above, further comprising the step of concluding on the pharmacological safety of the agent based on the detecting. This method also includes the result that in case of no substantial change of the luciferase activity as detected in the culture medium when compared to a genetically modified mammalian cell cultured without the agent to be screened for safety or a control cell the agent is usually regarded as pharmacologically unproblematic. The method then further comprises the step of concluding on the pharmacological safety of the agent to be tested or screened for safety based on the detecting as above.

Preferred is a method according to the present invention as above, wherein an increase of the luciferase activity as detected in the culture medium is indicative for an agent causing cellular damage, and thus being pharmacologically problematic, and wherein a decrease of the luciferase activity as detected in the culture medium is indicative for an agent causing cytoprotection, and thus being pharmacologically unproblematic.

The other details regarding this method are as described above and herein, in particular with respect to the cellular damage detection assay.

As above, preferred is a method according to the present invention, wherein the agent is selected from a pharmaceutically active compound, a drug, in particular a small molecule drug (i.e. less than 500 Da), an antibiotic, an antibody, a peptide, an immune cell, or an infectious agent, such as, for example, a bacterium, a fungus, or a virus. Examples for bacteria are intracellular bacteria such as *Yersinia.* Examples for fungal infections are *Candida* species (see also further below), and examples for viruses are Zika, HIV, coronavirus, RSV, HPV or West Nile Virus.

In a preferred embodiment of the method according to the present invention, genetically modifying the cell comprises transfection of the cell with a suitable vector, such as a recombinant lentiviral vector, encoding the luciferase in order to (stably) express the luciferase. Respective methods are described herein, and are known from the literature and to the person of skill in the art.

In a preferred embodiment of the method according to the present invention, the luciferase is a genetically modified luciferase, such as Nano-Luc (Nluc). Genetic variants (e.g. fusions or mutants) of Nluc may be used, as long as the important properties (size, stability, pH independence) of the enzyme in the method according to the present invention are not substantially impaired.

In a preferred embodiment of the method according to the present invention, detecting luciferase activity in the culture medium comprises optionally centrifuging down of the cell debris, collecting a suitable amount of supernatant from the culture, diluting the supernatant with a suitable buffer (e.g. PSA), adding luciferase substrate (e.g. coelenterazine), and, preferably immediately, measuring the luminescence as generated in a suitable device, such as a luminometer. Although not preferred, detection of luciferase may also be done using suitable antibody-tests, where the presence and/or amount of the Nluc enzyme is detected in the medium using suitable antibodies and detecting binding of said antibodies or antigen-binding fragments thereof.

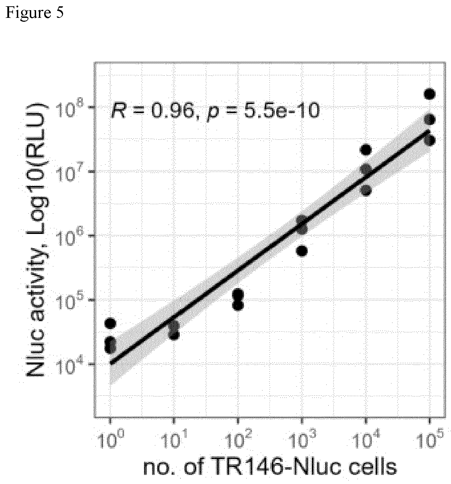

Another important aspect of the present invention then relates to method according to the present invention, further comprising at least one additional step of determining the number of cells in the culture medium based on determining the luciferase activity of the genetically modified cells as cultured, preferably in a lysate. As seen in for example Figure 1C and Figure 5, the number of cells correlates very well with Nluc-luminescence signal (linear correlation), and this way the determination/estimation of the absolute number of cells damaged in each sample is possible.

Another important aspect of the present invention then relates to the method according to the present invention which is, at least in part, automated. The inventive method can be performed by robots, and be upscaled using multiwell plates for culturing and testing, solid surfaces (e.g. chips or membranes) and the like.

Another important aspect of the present invention then relates to a diagnostic kit comprising materials for performing the method according to the present invention, in particular a luciferase substrate, a genetically modified mammalian cell line stably expressing Nluc, buffers, triton, positive controls, culture medium, optionally an agent to be tested, and/or instructions for use.

Another important aspect of the present invention then relates to the use of the diagnostic kit according to the present invention for detecting and/or determining cellular damage in a mammalian cell, for detecting and/or determining a cytoprotective agent in a damaged cell, for screening for an agent causing cytoprotection in a cell, or for determining the pharmacological safety of an agent according to the present invention as described herein.

In the context of the present invention a luciferase reporter system to measure cytotoxicity (damage) in mammalian epithelial cell lines in an infection model was designed and optimized. The inventors used a lentiviral system to transduce immortalized epithelial cells taken from niches, where invasive or superficial candidiasis typically occurs. This included the oral epithelial cell line TR146, vaginal A431, and intestinal C2BBe1, where the expression of luciferase may be indicated by the addition of -Nluc to the cell line name.

With the simple luciferase assay, the inventors were able to detect a measurable signal from as few as 100 TR146-Nluc cells. The Nluc cytotoxicity assays recapitulated the epithelial damage caused by *C*. *albicans* over the course of an infection, as observed by the popular LDH-based cytotoxicity assay. The inventors also tested the application for TR146-Nluc to detect mammalian cytotoxicity by different *Candida* species, *C*. *albicans* mutants, and the protective effects of antifungals. In all assays, the inventors found the Nluc data comparable, and in some cases even superior to the LDH assay. Finally, the inventors tested the stability of the Nluc and LDH enzymes in cell lysate over a range of temperatures, storage duration, and pH. The results as identified in the context of the present invention show that the inventive luciferase-based assay is a promising new tool for the detection of epithelial cell damage caused by *C*. *albicans* infections.

The Nluc cytotoxicity assay detects even low levels of cell damage quickly, reliably, and inexpensively, making it ideal for high-throughput applications.

After collecting the sample containing Nluc, and diluting it in a simple buffer, the step of adding freshly prepared luciferin can be easily automated, and the resulting luminescence can be measured almost immediately. This contrasts with the time-consuming steps involved in measuring LDH activity from supernatants of samples, which requires the fresh preparation of a complex stabilizing buffer and a catalyst, adding this mixture to the diluted samples, waiting 15-30 minutes, and then measuring the absorbance. After the initial cost of transducing cell lines, the assay is inexpensive and determined mostly by the cost of coelenterazine, or other luciferase substrates. Luciferase expressing cell lines created with non-proprietary luciferases (Matta et al., 2018) can further reduce the cost and be just as effective.

One limitation of using luminescence readouts is the lack of a protein standard to make standard curves, which correlate luminescence signal intensity to protein amount (ng/ ml) for each assay. Once the substrate luciferin is added, the reaction is instantaneous, and the luminescence signal decays over time. This leads to variability between independent replicates. Even with this limitation, in the present invention the inventors demonstrated that the Nluc-reporter cell lines can clearly distinguish damaged vs non-damaged samples (Figure 1B).

There are also other ways to overcome the variability between assays. One is to use an automatized substrate dispenser to minimize the time between addition of substrate and the measurement of luminescence. The inventors can also create a calibration curve for each assay, by lysing a fixed number of cells, serially diluting them, and measuring the luminescence. As seen in Figure 1C, the number of cells correlates very well with Nluc-luminescence signal, and this way the inventors estimate the absolute number of cells damaged in each sample.

The present invention provides a proof-of-concept for the Nluc cytotoxicity reporter assay for a well-studied fungal pathogen, *C*. *albicans.* Modifications of the assay, such as the addition of albumin (Pekmezovic M, et al. Human albumin enhances the pathogenic potential of Candida glabrata on vaginal epithelial cells. PLoS Pathog. 2021 Oct 28;17(10):e1010037. doi: 10.1371/journal.ppat.1010037. PMID: 34710198; PMCID: PMC8577789) allows the quantification of cell damage caused by non-albicans *Candida* species. Other fungal, bacterial, protozoan, or viral pathogens can be assayed with minor modifications to the protocol.

The extensive clinical use of antifungals to treat the increasing population at risk of *Candida* sp. infections, including people who are frequently hospitalized, the elderly, and the immunocompromised, have led to the emergence of drug resistance in *Candida* sp., particularly in *C*. *glabrata* (Pfaller et al., 2019). This, along with the urgent need to develop drugs to treat the emerging multi-drug resistant *C. auris*, has led to calls for expedited antifungal drug discovery (reviewed in (Ademe and Girma, 2020)).

Amongst the many practical challenges in developing new antifungal drugs (reviewed in (Roemer and Krysan, 2014)) a major one is the off-target toxicity of antifungal leads, because both, infecting fungi and human host are eukaryotes, and many potential fungal targets are conserved between them. Most drug discovery efforts focus on first assaying antimicrobial activity in *in vitro* growth assays, and then study the side effects of the selected hits on human cells. The inventors found that the Nluc-reporter cell line was sensitive in detecting protection from *C. albicans*-induced epithelial damage in the presence of subinhibitory levels of antifungals (Figure 1D). This demonstrates a promising application for the Nluc-reporter cell lines, that can be easily adapted to a one-step assay with simultaneous readouts for both mammalian cytotoxicity using the Nluc assay, and antimicrobial effects using reporter microbe strains that quantify microbial biomass with microscopy (for example *Candida*-GFP). In this way, a host cell cytotoxicity reporter can expedite the discovery of non-toxic anti-microbial drugs and antivirulence drugs.

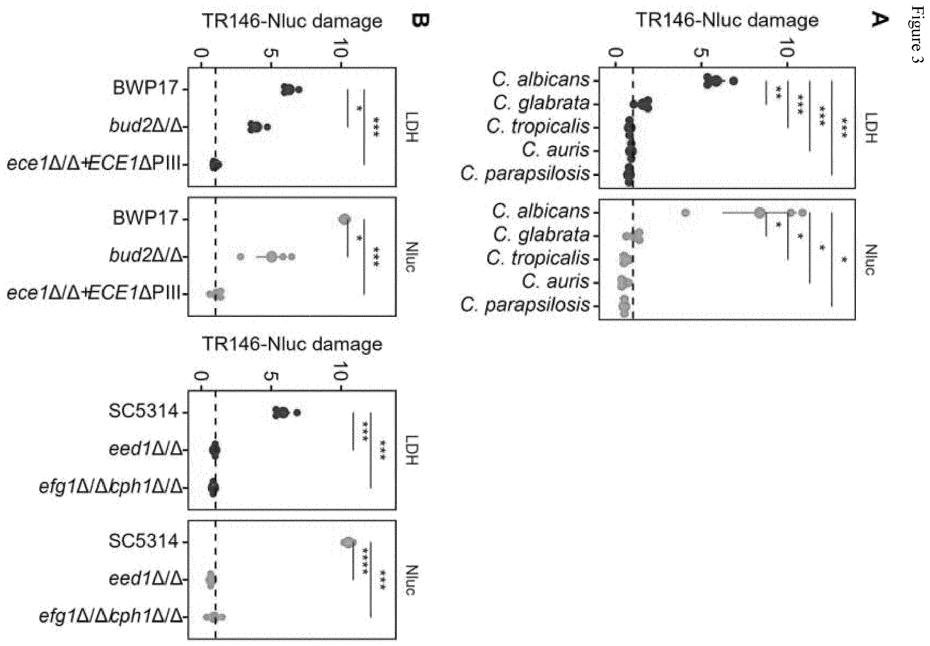

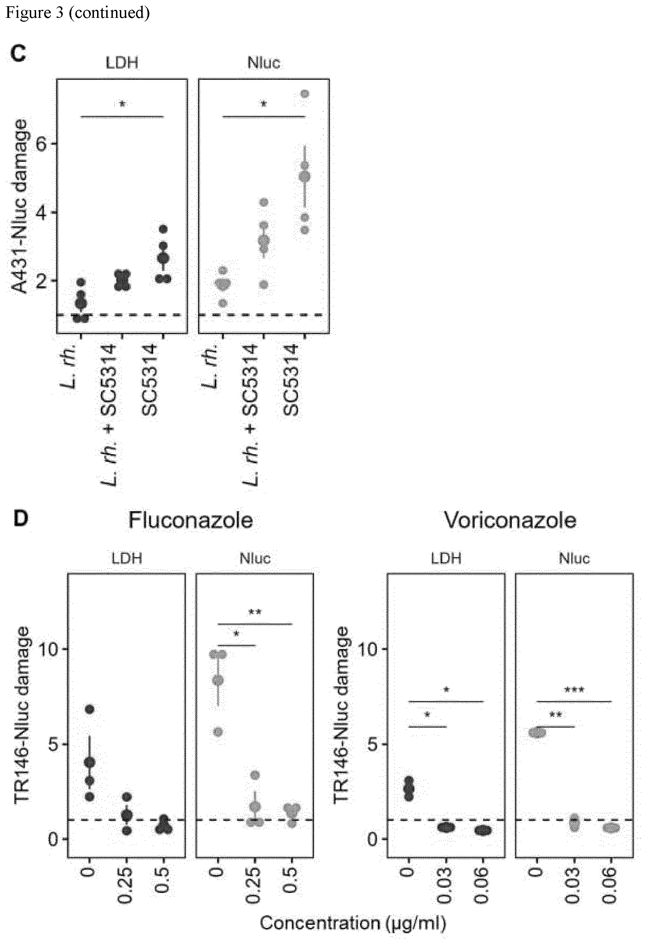

The public-health risks have galvanized research and discovery in the *Candida* field, such that now, *C*. *albicans* is a genetically tractable organism with large collections of gene knock-out libraries (Homann et al., 2009; Noble et al., 2010), well characterized *in vitro* models with a clear host-cell-damaging phenotype (Last et al., 2021), and extensively studied antifungal drug interactions (Cowen et al., 2015) (Figure 3). The Nluc damage reporter can just as easily be applied to study damage to cell-types induced by any microorganism, drugs, noxious substances, and other host cells.

The inventors also found that the lentiviral transduction system was easily adaptable for creating multiple Nluc-reporter cell lines. After engineering the appropriate plasmids and harvesting virus-like particles from transfected HEK293 cells, the viral particles could be stored at -80°C for an extended period and used to create and select new Nluc cell lines as needed, requiring minimal effort.

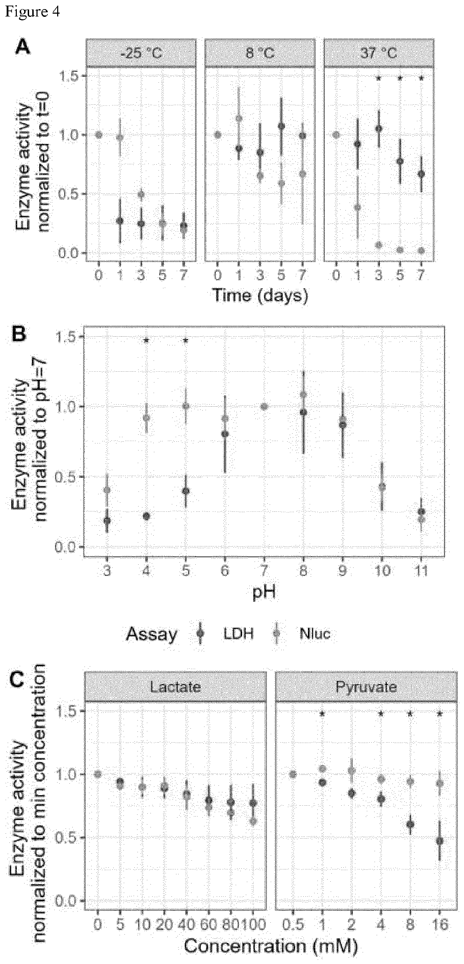

The inventive system and method have several advantages and strengths when using the Nluc damage-reporter in place of the conventional LDH cytotoxicity assay. For instance, certain bacteria lower the pH of cell culture medium when co-cultured with human cells, and in these cases, LDH release assays underestimate mammalian-cell damage because the lowered pH inhibits LDH (Gay et al., 1968; Van den Bossche et al., 2020). Given Nluc's stability over a wide pH range (Figure 4B, (Hall et al., 2012)), an Nluc-reporter cell lines will be particularly useful in assaying cell damage in such models, and better mimic physiologically relevant niches of low pH (Lindén et al., 2007).

Furthermore, the present Nluc-reporter detects damage specific to transduced cells. LDH, an enzyme found all across the kingdom of life, catalyzes pyruvate to lactate in many pathogens, including some fungi (Gleason et al., 1966; Lockwood, L.B., 1936), many bacteria (Garvie, 1980), and protozoa (Dunn et al., 1996; Makler et al., 1998). Therefore, when developing infection models to study non-model organisms, it is important to use damage assays that do not rely on LDH. Because of these concerns, some researchers have relied on ⁵¹Cr release to measure mammalian cell damage inflicted for example by *C. albicans* and *Rhizopus delemar* (Fukuoka et al., 2003; Soliman et al., 2021). However, the use of radioactive materials comes with its own dangers and difficulties and requires specialized training and facilities that are not easily accessible. This makes Nluc-reporter cell lines an attractive alternative for assaying host-cell-specific damage.

Luciferase-expressing cells have also been recently used to quantify damage to transduced cells in models that contain a mixture of mammalian cells. For example, to study quantify the cytotoxicity of chimeric antibody expressing immune cells that kill luciferase-transduced cancer cells (Matta et al., 2018) and to screen antibodies from patient blood that mediate antibody-dependent cellular toxicity towards cancer cells (Vincken and Ruiz-Saenz, 2023). This approach has also been used to identify small molecules that modulate NK cells to kill luciferase-transduced cancer cells more readily (Cortés-Kaplan et al., 2021). In the context of the present invention, the inventors tested applications for epithelial-Nluc in 2D cell culture models. Historically, these models have provided molecular insights into commensal and pathogenic interactions of fungi with specific cell types (reviewed in (Last et al., 2021)).

Finally, the Nluc-reporter cell lines can be used in organ-on-chip, trans-well, or similar more complex models that include immune cells - which are key players in host-pathogen interactions (Last A, et al. In vitro infection models to study fungal-host interactions. FEMS Microbiol Rev. 2021 Sep 8;45(5):fuab005. doi: 10.1093/femsre/fuab005. PMID: 33524102; PMCID: PMC8498566). Here, the luciferase-reporter cell lines are able to distinguish between damage to immune and epithelial components.

To summarize, the inventors developed a luciferase-based assay to detect cytotoxicity in mammalian epithelial cell lines. The assay is simple, reliable, inexpensive, and can be used to detect even low levels of cell damage. It is also more specific than the traditional lactate dehydrogenase (LDH) assay, as it can distinguish between damage to transduced cells and damage to other cells in the culture. The inventors tested the Nluc-reporter cell lines extensively in *C*. *albicans* infection models and found that the assay could detect the damage caused by the pathogen, and that it was comparable to or even better than the LDH assay.

While *C. albicans* served as a good model and case study, the inventors' data shows that the assay is applicable with minimal changes to test other fungal, bacterial, viral, or protozoan infections as well as a range of cell damaging substances. The inventors also tested the stability of the assay over a range of temperatures, storage durations, and pH. The inventors found that the assay was stable under a wide range of conditions, making it suitable for a variety of applications. Overall, the present results suggest that the luciferase-based assay is a promising new tool for the detection of cytotoxicity in mammalian epithelial cell lines.

The present invention relates to the following items:
Item 1. An *in vitro* method for detecting and/or determining an agent causing cellular damage in a eukaryotic, in particular, mammalian cell, comprising the steps of
   a) providing a eukaryotic, in particular, mammalian cell that is genetically modified to stably express an intracellular luciferase enzyme,
   b) contacting the cell of a) with at least one agent suspected to cause cellular damage,
   c) suitably culturing the cell of b) in a culture medium, and
   d) detecting luciferase activity in the culture medium,
   wherein a change of the luciferase activity as detected in the culture medium when compared to a genetically modified mammalian cell that was not contacted with the at least one agent is indicative for an agent causing cellular damage in the mammalian cell.
Item 2. An *in vitro* method for detecting and/or determining a cytoprotective agent for a eukaryotic, in particular mammalian cell, comprising the steps of
   a) providing a eukaryotic, in particular, mammalian cell that is genetically modified to stably express an intracellular luciferase enzyme,
   b) contacting the cell of a) with at least one first agent causing cellular damage,
   b) suitably culturing the cell of a) in a culture medium,
   c) contacting the agent to be tested with the cell as cultured in b), and optionally suitably culturing the cells in a culture medium
   d) detecting luciferase activity in the culture medium, and
   wherein a change of the luciferase activity as detected in the culture medium when compared to a genetically modified mammalian cell cultured without the agent to be tested is indicative for cytoprotection in the mammalian cell caused by the agent.
Item 3. The method according to Item 1 or 2, wherein an increase of the luciferase activity as detected in the culture medium is indicative for an agent causing cellular damage, and wherein a decrease of the luciferase activity as detected in the culture medium is indicative for an agent causing cytoprotection.
Item 4. The method according to any one of Items 1 to 3, further comprising the step of concluding on the pharmacological safety of the agent based on the detecting.
Item 5. The method according to any one of Items 1 to 4, wherein the agent causing cellular damage is selected from agents related to senescence, infection, environmental factors, cytotoxicity, cancer, radiation or physical damage, a pharmaceutically active compound, a drug, in particular a small molecule drug, an antibiotic, an antibody, a peptide, an immune cell.
Item 6. The method according to Item 5, wherein the cell is infected with an infectious agent, such as, for example a bacterium, a fungus, or a virus, such as, for example, *Candida, Yersinia,* Zika, HIV, Coronavirus, RSV, or West Nile Virus.
Item 7. The method according to any one of Items 1 to 6 wherein the eukaryotic cell is selected from the group consisting of insect cells, fungal cells, e.g., yeast, and mammalian cell selected from the group consisting of epithelial cells, vaginal cells, intestinal cells, lung cells, immortalized cells, cancer cells, and cell lines thereof.
Item 8. The method according to any one of Items 1 to 7, wherein genetically modifying the cell comprises transfection of the cell with a recombinant lentiviral vector encoding the luciferase.
Item 9. The method according to any one of Items 1 to 8, wherein the luciferase is a genetically modified luciferase, such as Nano-Luc (Nluc).
Item 10. The method according to any one of Items 1 to 9, wherein detecting luciferase activity in the culture medium comprises optionally centrifuging down of the cell debris, collecting a suitable amount of supernatant, diluting the supernatant with a suitable buffer, adding luciferase substrate, and measuring the luminescence as generated.
Item 11. The method according to any one of Items 1 to 10, further comprising at least one additional step of determining the number of cells in the culture medium based on determining the luciferase activity of the genetically modified cells as cultured preferably in a lysate.
Item 12. The method according to any one of Items 1 to 11, which is, at least in part, automated.
Item 13. A diagnostic kit comprising materials for performing the method according to any one of Items 1 to 12, in particular a luciferase substrate, a genetically modified mammalian cell line stably expressing Nluc, buffers, triton, positive controls, culture medium, optionally an agent to be tested, and/or instructions for use.
Item 14. Use of the diagnostic kit according to Item 13 for detecting and/or determining cellular damage in a mammalian cell, for detecting and/or determining a cytoprotective agent in a damaged cell, for screening for an agent causing cytoprotection in a cell, or for determining the pharmacological safety of an agent according to any one of Items 1 to 12.

The invention will now be described further in the following examples with reference to the accompanying figures, nevertheless, without being limited thereto. For the purposes of the present invention, all references as cited are incorporated by reference in their entireties.
Figure 1 shows that Nluc is stably expressed in transduced epithelial cells. A. Schematic representation of lentiviral transduction to generate Nluc epithelial cell lines. B. Cell lysates from TR146-Nluc were collected over three passages, solubilized for SDS-PAGE, blotted and proteins detected with the indicated antibodies. Representative image of two independent replicates shows that TR146-Nluc cells are stably expressing Nluc. C. Relationship between luminescence of cell lysate and the number of TR146-Nluc cells that were lysed. All experiments were performed in 2-3 independent replicates, where the white circles represent value from each replicate. Standard deviation about the mean (black circle) is represented as black lines. Student's t-test was used to compare the means, where *, **, *** denote p<0.05, 0.01, 0.001, respectively.

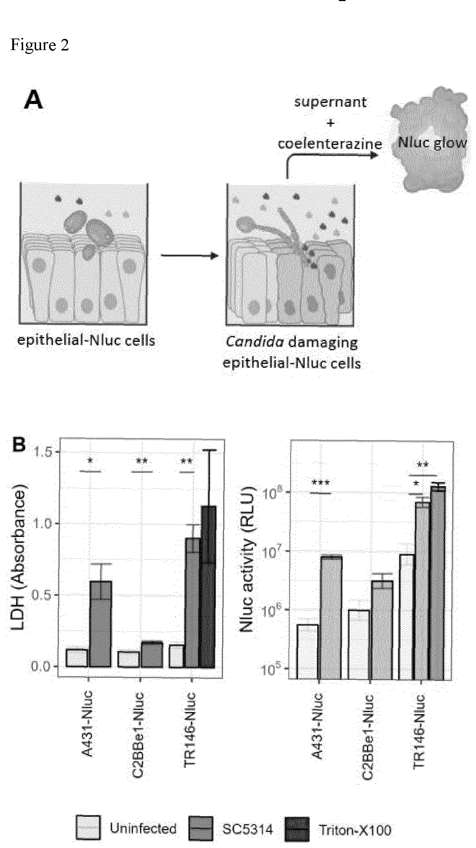

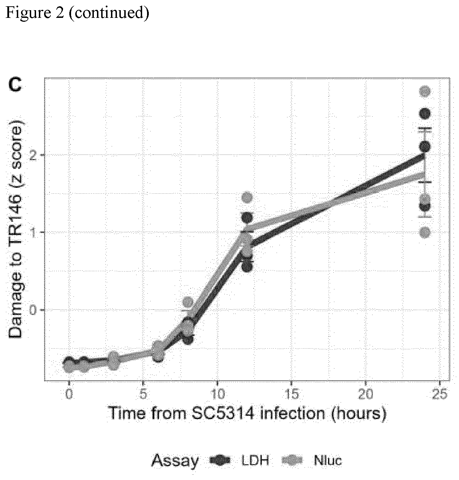

Figure 2 shows that the inventive Nluc luminescence assay reflects C. *albicans* (SC5314)-induced epithelial damage. A. Scheme of Nluc-damage assay depicting transduced epithelial Nluc cells infected with *C*. *albicans. C. albicans* invades and damages epithelial cells, releasing cytoplasmic proteins Nluc (dark globules) and LDH (gray globules) into the supernatant. The supernatant is collected and diluted for the assay. The luciferase substrate coelenterazine is added to the diluted supernatant, which leads to emission of light that is measured by a luminometer. B. Damage to Nluc-expressing vaginal (A431), gut (C2BBe1) or oral (TR146) epithelial cells infected with *C*. *albicans* was compared to uninfected cells, or cells lysed by Triton X-100, by measuring LDH and Nluc activity of supernatant collected 24 hours after infection. Bars depict mean LDH or Nluc activity (log scaled), error bars depict standard deviation about mean. Infected vs uninfected were compared with Student's t-test, with *, p<0.05 and **, p<0.01. All experiments were carried out in three independent replicates. C. Damage to oral epithelial (TR146-Nluc) cells was estimated by Nluc (light gray) and LDH (dark gray) assays at different time points after infection with *C*. *albicans* (x-axis). Values on the y-axis are presented as Z scores, i.e., they are centered around their respective mean, to allow direct comparison of the two assays. Error bars represent standard deviation about the mean. All experiments were carried out in three independent replicates.
Figure 3 shows examples for applications of the inventive epithelial cells Nluc damage assays: A. Damage to TR146-Nluc cells by different *Candida* species. B. In both, LDH and Nluc assay *C*. *albicans* virulence factor deletion mutants show reduced damage in TR146-Nluc assays compared to their background strains BWP17 and SC5314. C. In a commensal vaginal epithelial model, *L. rhamnosus* (L. rh.) protects A431-Nluc cells from damage against *C*. *albicans* infection. D. Damage to TR146-Nluc cells infected with *C*. *albicans* (SC5314) at sub-MIC fluconazole and voriconazole concentrations. Damage to epithelial cells, estimated by LDH and Nluc assays, was normalized to uninfected controls (dotted line). Experiments were performed in two to three independent replicates, with three technical replicates each. The error bars represent standard deviation about the mean. A t-test with was applied, where *, **, ***, **** denote p<0.05, 0.01, 0.001, and 0.0001, respectively.
Figure 4 shows limitations and strengths of the Nluc cytotoxicity assay: A. Nluc and LDH activity of cell lysate incubated at -25, 8 and 37°C for 0-7 days. B. Nluc and LDH activity of cell lysate incubated with PBS buffered at various pH values. C. Nluc and LDH activity of cell lysates incubated with increasing concentrations of lactate and pyruvate. D. Experiments were performed in three independent replicates, with three technical replicates each. Error bars represent standard deviation about the mean, where the mean is represented as a point. LDH vs Nluc were compared with Student's t-test, where * denotes p<0.05.
Figure 5 shows a scatter plot depicting the correlation (Pearson's correlation) between luminescence of cell lysate and the number of TR146-Nluc cells that were lysed (the same data was visualized figure 1C). The axes are log10 transformed. Standard deviation about the mean is represented as black lines. All experiments were performed in 2 to 3 independent replicates.

### Examples

### Materials and methods

### Candida strains and cultivation

**Table 1 Candida strains used in the present invention**

| **Species** | **Strain name** | **Genotype** | **Internal ID** | **Reference** |
|---|---|---|---|---|
| *C. albicans* | SC5314 | Genome sequence reference strain | C55 | (Gillum et al., 1984) |
| | BWP17+CIp30 | BWP17, *rps1*::*(HIS1 ARG4 URA3)* | M130 | (Fonzi and Irwin, 1993) |
| *C. glabrata* | ATCC 2001 | Genome sequence reference strain | C94 | (Li et al., n.d.) |
| *C*. *parapsilosis* | GA1 | *C. parapsilosis* WT strain GA1 | C118 | (Gácser et al., 2007) |
| *C. tropicalis* | DSM 4959 | *C. tropicalis* WT strain DSM 4959 | C30 | (Borg et al., 1984) |
| *C. albicans* | *efg1*ΔΔ\*cph1*ΔΔ | SC5314, *cph1*::FRT1/*cph1*::FRT1 *efg1*::FRT/*efg1*::FRT | M2188 | (Lo et al., 1997) |
| | *eed1*ΔΔ | SC5314, *eed1*::FRT/*eed1*::(FRT-SAT1-FRT) | M1314 | (Wartenberg et al., 2014) |
| | *bud2*ΔΔ | BWP17, *bud2*::*ARG4*/p*URA3-BUD2*::*bud2*::*HIS1* | M1411 | (Hausauer et al., 2005) |
| | *ece1-III*ΔΔ | BWP17, *ece1*::*HIS1*/*ece1*::*ARG4* rps1::(*URA3 ECE1*^{ΔPIII}) | M2174 | (Moyes et al., 2016) |

| | | | | |
|---|---|---|---|---|
| *Candida* sp. strains, cryopreserved at -80°C, were streaked onto YPD-agar plates and incubated at 37°C for 24 hours. Overnight cultures were prepared by inoculating single colonies into YPD 1% peptone in Erlenmeyer flasks for incubation at 30 °C, 180 rpm. | | | | |

### Molecular biology, lentiviral transfection, and transduction

The Nluc gene was amplified with "pCRISPaint-NanoLuc" plasmid purchased from Addgene (Veit Hornung Addgene plasmid #67178) and ligated to the lentiviral transfer plasmid pSEW-NanoLuc-IRES-PuroR, The final reporter vector was confirmed via vector digestion with additional sequencing of the reporter sequence "Nluc_IRES_PuroR".

For the generation of lentivirus-like particles, HEK-293T/17 cells were transfected with a mixture of the transfer plasmid pSEW - NanoLuc - IRES _PuroR and helper plasmids psPAX2 (Addgene plasmid #12260) and pMD2G (Addgene plasmid #12259), combined in jetPRIME transfection agent buffer, as per manufacturer's protocol. Lentiviral supernatant was filtered and concentrated with the Lenti-X concentrator as per the manufacturer's protocol (Clontech Labs). A431, TR146 and C2BBe1 cells were transduced by infecting cells with lentiviral particles for 48 hours, and selecting them in puromycin selection medium for 10-14 days. Selected cells were passaged and cryo-preserved in 20% DMSO + heat inactivated FBS.

### Mammalian cells lines and cell culture.

**Table 2: Mammalian cell culture**

| Cell line name | Cell line description | Genotype | Medium used | Reference |
|---|---|---|---|---|
| TR146 | human buccal (oral) epithelial cell line | Wild type | DMEM: Ham's F12 medium (1:1) | ECACC (from Sigma Aldrich), Lot No. 13D016 |
| C2BBe1 | human duodenum adenocarcinoma | Wild type | DMEM + 10 µg/ml human holo transferrin | ATCC (CRL-2102), Lot: 61034680 |
| A431 | human vaginal epithelial cell line | Wild type | RPMI 2 g/l glucose | DSMZ |

Mammalian cells were cultured and passaged in their respective medium (table 2), supplemented with 10% FBS in 75 cm² tissue culture flasks. Confluent TR146 and A431 cells were detached using Accutase for 15-20 minutes, while C2BBe1 cells were detached using RPMI + Trypsin for 10 minutes. Detached cells were resuspended in 3 times the volume of their respective medium supplemented with 10% FBS (table 3) and counted using a Neubauer counting chamber. A cell suspension containing 10⁵ cells/ ml was prepared, and 200 µl of cell suspension was distributed evenly into a 96-well plate. Cells were incubated at 37 °C, 5% CO₂ for ≈ 2 days.

### Western blot to confirm stability of luciferase expression.

To confirm the stability of Nluc expression across passages of transduced cells, cells from consecutive passages were collected, centrifuged at 10,000 × g and the pellet stored at -20 °C. Cells were lysed in Radio-Immunoprecipitation Assay (RIPA) buffer (Merck) with protease inhibitor cocktail (Calbiochem), and Benzonase (25 U/µl, (Novagen). Proteins were quantified by bicinchoninic acid assay, and run on an SDS PAGE (12% separating, 5% stacking) in Laemmli buffer. Protein bands were transferred to nitrocellulose membranes and incubated with monoclonal anti-Nanoluc antibody (Promega) and monoclonal anti-beta Tubulin antibody (AbD Serotec). Proteins were visualized using anti-mouse HRP-conjugated secondary antibody (Pierce) with an Odyssey M imaging system (LI-COR Biosciences) and images were processed in Image Studio (LI-COR Biosciences).

### Epithelial cell damage assays.

In order to measure damage from cells seeded in 96-well plates, plates were centrifuged at 250 × *g* for 10 minutes. Where indicated, 100 µl of supernatant was collected and stored at -20 °C for assaying on another day in the same week. For the luciferase assays, 5 µl supernatant was diluted with 95 µl PBS in white flat-opaque-bottomed 96-well plates (ThermoFisher). Then, 100 µl of 10 µM coelenterazine (in PBS) was added and luciferase activity measured immediately using a Tecan infinite M200 luminescence reader, with integration time of 100 ms and OD2 compensation. The OD2 neutral density filter attenuates high level light by a factor of 100.

The LDH cytotoxicity assay was performed according to instructions in the Roche Cytotoxicity Detection Kit with minor modifications. Briefly, 10 µl supernatant was diluted with 90 µl PBS in transparent-flat-bottomed 96-well plates, 100 µl of kit reagent mixture was added to each well, the plates were incubated in the dark for 15-20 minutes at room temperatures, and absorbance was measured with a Tecan absorbance reader at 492 nm (reference at 600 nm).

### Quantifying Nluc activity per TR146-Nluc cell from lysate

TR146-Nluc cells were detached with Accutase, resuspended in PBS, and counted with a Neubauer counting chamber. A dilution of 2 × 10⁶ cells/ml was prepared in PBS, which was serially diluted 1:10 till 2 × 1¹⁰ cells/ml in lysis buffer (1% Triton X-100 in PBS), and incubated at 37°C for 30 minutes. 100 µl of 10 µm coelenterazine was added to 100 µl of cell lysate, such that the inventors measured Nluc activity ranging from ≈ 1 to ≈ 10⁵ cells.

### Infection of epithelial cells with Candida spp. strains

Overnight cultures of *Candida* spp. strains in YPD, 1 % peptone were diluted, washed in PBS, and counted using Neubauer counting chambers. The medium of confluent epithelial cells was replaced with a suspension of 1 × 10⁵ cells/ml *Candida* spp. (MOI 1) in the appropriate cell culture medium (without FBS). The cells were incubated at 37 °C and 5% CO2 for 24 hours.

### Time course of damage to epithelial cells induced by C. albicans

TR146-Nluc cells were seeded into 96-well plates, infected with C. *albicans* (SC5314) at MOI 1, as per the protocol detailed above, and supernatants were collected from separate wells at 0, 3, 8, 12 and 24 hours and stored at -20°C. Samples were thawed the next day, centrifuged (250 × g), and assayed for luciferase and LDH activity.

### Infection of epithelial cells with C. albicans in the presence of antifungals

Stock solutions of the antifungal drugs, fluconazole (10 mg/ml) and voriconazole (10 mg/ml) were prepared in DMSO and stored at -20 °C. A two-fold dilution series was prepared in DMEM/F12 cell culture medium for fluconazole (8-0.25 µg/ml) and voriconazole (1-0.3125 µg/ml). Diluted drugs were transferred to TR146-Nluc cells seeded 48 hours prior, and infected with C. *albicans* (SC5314), as described above. Supernatant was collected 24 hours after infection to measure damage by LDH and Nluc.

### L. rhamnosus colonization and C. albicans infection in vitro vaginal epithelial model

*L. rhamnosus* ATCC 7469 was cultured in MRS Broth for 48 hours at 37 °C with 5% CO₂ and 1% O₂. *L. rhamnosus* was washed in PBS and diluted in RPMI-1640 to optical density 0.2 (OD₆₀₀). For *L. rhamnosus* colonization, 16-18 hours prior to *C*. *albicans* infection, the medium of A431-Nluc was replaced with 50 µl of a suspension of *L. rhamnosus* in RPMI-1640. For fungal infection, 50 µl of *C*. *albicans* suspension (MOI 1) was prepared in RPMI-1640 and added to the colonized cells. After 24 hours, supernatant was collected for luciferase and LDH assays.

### Robustness of Nluc and LDH activity at different pH, temperature, and pyruvate concentrations

TR14-Nluc cells were detached with Accutase and resuspended in 5 ml PBS. Cells were counted and ≈ 1 × 10⁴ cells/ml were resuspended in lysing buffer (1% Triton X-100 in PBS, pH 7.4).

For the effect of different pH on the enzyme activities, the lysis buffer was adjusted to pH 3, 4, 5, 7, 8, 9, 10 or 11 with HCl or NaOH, and this buffer was used to resuspend cells, which were subsequently incubated for 10 minutes at 37 °C, centrifuged at 10,000 × *g* to remove cell debris, and supernatant was collected to measure LDH and luciferase activity.

In order to test the effect of storage at different temperatures over time, the cell lysates were incubated for 10 min at 37 °C, centrifuged at 10,000 × *g*, and supernatant aliquots were stored at 37 °C, room temperature (≈28 °C), 8 °C or -20 °C. One aliquot was thawed every other day to measure luciferase and LDH activity.

To examine the effects of pyruvate on activity of LDH, a 2-fold dilution series for sodium pyruvate and lactate (16-0.5 mM) was prepared in lysis buffer, the lysate was incubated for 10 min at 37 °C and centrifuged at 10,000 × *g*. The supernatant was collected to test LDH and luciferase enzyme activity.

### Luciferase activity from lysates of stably transduced epithelial Nluc cells correlates with the number of cells

The inventors transduced TR146, C2BBe1, and A431 cell lines with lentiviral particles containing an Nluc insert with a constitutively active promoter and a puromycin selection cassette. To confirm that TR146-Nluc cells stably expressed Nluc over time, the inventors collected cells from different passages, lysed them, and performed western blot with anti-Nluc antibody (Figure 1B). The stable expression of Nluc in C2BBe1-Nluc and A431-Nluc was similarly confirmed by western blot. The inventors also noted that luciferase activity highly correlated with the number of cells lysed, and it reliably detected as few as 100 cells (Figure 1C and Figure 5).

### Epithelial Nluc cell lines function as cytotoxicity reporters in C. albicans infection models

The inventors infected TR146-Nluc, C2BBe1-Nluc, and A431-Nluc cells with *C. albicans* (genomic reference wild type strain for *C*. *albicans,* SC5314) for 24 hours and measured the Nluc activity in the supernatant. *C*. *albicans-infected* cells released significantly more luciferase than uninfected cells (Figure 2B), showing that the present system can detect fungal-induced epithelial cell damage. The Nluc cytotoxicity reporter assay can be performed simply by collecting 5-10 µl supernatant after spinning down the cell debris, diluting the supernatant to 100 µl with PBS, adding the luciferin, and immediately measuring the luminescence (Figure 2A).

Next, the inventors wanted to verify that the luciferase reporter cell line TR146-Nluc can recapitulate the temporal dynamics of a *C*. *albicans* infection as well as the popular cytotoxicity assay, LDH. To this end, the inventors collected supernatants from infected TR146-Nluc cells at various timepoints up to 24 hours, and found that the readouts from LDH and luciferase assays were highly in agreement over the entire time course (Figure 2C).

### Applications for Nluc-epithelial cytotoxicity reporter in C. albicans in vitro infection models

Next, the inventors tested the Nluc-based cytotoxicity reporter for typical applications in *C*. *albicans* infection biology and compared the luciferase readouts with the conventional LDH assay. As reported previously for such *in vitro* models, the filamenting *C*. *albicans* caused significant damage to monolayers of oral, vaginal, and gut epithelial cells, but other *Candida* spp. including *C. glabrata, C. auris*, and *C. parapsilosis* did not elicit detectable host cell damage in LDH and Nluc assays (Figure 3A). C. *albicans* mutants with deletions for genes or sequences encoding the peptide toxin candidalysin (*ECE1-PIII*) (Moyes et al., 2016), transcriptional regulators of filamentation (*EFG1*/*CPH1* or *EED1*) (Lo et al., 1997; Martin et al., 2011; Riggle et al., 1999) or thigmotropism *(BUD2)* (Hausauer et al., 2005) are defective in virulence to different extents. They were attenuated in damaging TR146-Nluc cells (Figure 3B), and the amount of damage reduction agreed well with the LDH assays done in parallel and with the phenotypes known from literature.

In summary, these experiments show that the present epithelial-Nluc cell assay agrees well with the conventional and widely used LDH detection system, and that it can be reliably used to screen and test different *C*. *albicans* strains and mutants.

Probiotics like *Lactobacillus rhamnosus* have recently garnered interest due to their ability to attenuate *C*. *albicans* virulence (Alonso-Roman et al., 2022; Graf et al., 2019). The inventors asked if *L. rhamnosus* colonization could protect A431-Nluc cells from *C. albicans-induced* damage. The inventors observed a clear trend towards reduced damage induced by *C*. *albicans* alone versus damage caused in combination with *L. rhamnosus,* although these differences were not statistically significant (Figure 3C). This potential reduction was seen in both assays, LDH and Nluc.

Next, the inventors tested whether commonly used antifungals - fluconazole and voriconazole - can protect TR146-Nluc from *C. albicans*-induced cell damage, and found that with Nluc, but not with LDH, the inventors were able to reliably detect the protective effect of antifungals even at sub-MIC levels (Figure 3D).

### Robustness of Nluc and LDH activity at different pH, temperature, and pyruvate concentrations

Supernatants from cell damage assays can be used immediately to measure cytotoxicity, or they can be stored for a while before measurement. To determine how long, and at what temperatures, Nluc and LDH activities are stable and accurate measures of cell damage, the inventors stored TR146-Nluc cell lysate at -25°C, 8°C, and 37°C. The inventors measured the Nluc and LDH activities from aliquots every other day over a week. The Nluc activity declined steeply on the third day of storing the supernatant at -25°C, while LDH activity was already mostly lost directly after freezing (Figure 4A). Nluc and LDH activities remained mostly stable at 8°C. At 37°C, Nluc activity was lost over the first days, while LDH remained mostly stable up to a week. Therefore, supernatants collected from epithelial-Nluc cells should be used to detect luciferase activity either immediately or can be stored at 8°C for up to a week or at -25°C for up to a day.

Infection of human cells often changes the pH of cell culture medium due to the metabolic activities of the microorganisms. The inventors therefore tested the pH range at which Nluc (or LDH) activity is a reliable measure of cytotoxicity by diluting cell lysate in PBS buffered at different pH values, and then assaying LDH and Nluc activity (Gay et al., 1968). The inventors found that Nluc out-performed LDH and gave consistent readings from pH 4 to 9 (Figure 4B), while the LDH assay was highly pH-dependent. Lastly, the inventors examined the effect of the known LDH inhibitor pyruvate, which is also a common cell culture medium component, on Nluc, and found no discernible effect on Nluc enzyme activity (Figure 4C).

### References as cited

Ademe, M., Girma, F., 2020. Candida auris: From Multidrug Resistance to Pan-Resistant Strains. Infect. Drug Resist. 13, 1287-1294. https://doi.org/10.2147/IDR.S249864
Allert, S., Förster, T.M., Svensson, C.-M., Richardson, J.P., Pawlik, T., Hebecker, B., Rudolphi, S., Juraschitz, M., Schaller, M., Blagojevic, M., Morschhäuser, J., Figge, M.T., Jacobsen, I.D., Naglik, J.R., Kasper, L., Mogavero, S., Hube, B., 2018. Candida albicans-Induced Epithelial Damage Mediates Translocation through Intestinal Barriers. mBio 9, e00915-18. https://doi.org/10.1128/mBio.00915-18
Alonso-Roman, R., Last, A., Mirhakkak, M.H., Sprague, J.L., Möller, L., Großmann, P., Graf, K., Gratz, R., Mogavero, S., Vylkova, S., Panagiotou, G., Schäuble, S., Hube, B., Gresnigt, M.S., 2022. Lactobacillus rhamnosus colonisation antagonizes Candida albicans by forcing metabolic adaptations that compromise pathogenicity. Nat. Commun. 13, 3192. https://doi.org/10.1038/s41467-022-30661-5
Baki, A., Bielik, A., Molnár, L., Szendrei, G., Keseru, G., 2007. A High Throughput Luminescent Assay for Glycogen Synthase Kinase-3 β Inhibitors. Assay Drug Dev. Technol. 5, 75-83. https://doi.org/10.1089/adt.2006.029
Borg, M., Kirk, D., Baumgarten, H., Rüchel, R., 1984. A colorimetric assay for the assessment of cytotoxicity of yeasts. Sabouraudia 22, 357-367. https://doi.org/10.1080/00362178485380611
Brown, G.D., Denning, D.W., Gow, N.A.R., Levitz, S.M., Netea, M.G., White, T.C., 2012. Hidden killers: human fungal infections. Sci. Transl. Med. 4, 165rv13. https://doi.org/10.1126/scitranslmed.3004404
Brunke, S., Mogavero, S., Kasper, L., Hube, B., 2016. Virulence factors in fungal pathogens of man. Curr. Opin. Microbiol., Host-microbe interactions: parasites/fungi/viruses 32, 89-95. https://doi.org/10.1016/j.mib.2016.05.010
Case, N.T., Duah, K., Larsen, B., Wong, C.J., Gingras, A.-C., O'Meara, T.R., Robbins, N., Veri, A.O., Whitesell, L., Cowen, L.E., 2021. The macrophage-derived protein PTMA induces filamentation of the human fungal pathogen Candida albicans. Cell Rep. 36, 109584. https://doi.org/10.1016/j.celrep.2021.109584
Cortés-Kaplan, S., Hasim, M.S., Kaczmarek, S., Taha, Z., Maznyi, G., McComb, S., Lee, S.-H., Diallo, J.-S., Ardolino, M., 2021. A small molecule drug screening identifies colistin sulfate as an enhancer of Natural Killer cell cytotoxicity. https://doi.org/10.1101/2021.08.20.457155 Cowen, L.E., Sanglard, D., Howard, S.J., Rogers, P.D., Perlin, D.S., 2015. Mechanisms of Antifungal Drug Resistance. Cold Spring Harb. Perspect. Med. 5, a019752. https://doi.org/10.1101/cshperspect.a019752
Cox, M.C., Mendes, R., Silva, F., Mendes, T.F., Zelaya-Lazo, A., Halwachs, K., Purkal, J.J., Isidro, I.A., Félix, A., Boghaert, E.R., Brito, C., 2021. Application of LDH assay for therapeutic efficacy evaluation of ex vivo tumor models. Sci. Rep. 11, 18571. https://doi.org/10.1038/s41598-021-97894-0
Dubois, R., 1885. Note sur la fonction photogenique chez les Pholades. Impr. G. Rougier. Dunn, C.R., Banfield, M.J., Barker, J.J., Higham, C.W., Moreton, K.M., Turgut-Balik, D., Brady, R.L., Holbrook, J.J., 1996. The structure of lactate dehydrogenase from Plasmodium falciparum reveals a new target for anti-malarial design. Nat. Struct. Biol. 3, 912-915. https://doi.org/10.1038/nsb1196-912
England, C.G., Ehlerding, E.B., Cai, W., 2016. NanoLuc: A Small Luciferase is Brightening up the Field of Bioluminescence. Bioconjug. Chem. 27, 1175. https://doi.org/10.1021/acs.bioconjchem.6b00112
Fonzi, W.A., Irwin, M.Y., 1993. Isogenic Strain Construction and Gene Mapping in Candida Albicans. Genetics 134, 717-728.
Fraga, H., 2008. Firefly luminescence: a historical perspective and recent developments. Photochem. Photobiol. Sci. Off. J. Eur. Photochem. Assoc. Eur. Soc. Photobiol. 7, 146-158. https://doi.org/10.1039/b719181b
Fukuoka, T., Johnston, D.A., Winslow, C.A., de Groot, M.J., Burt, C., Hitchcock, C.A., Filler, S.G., 2003. Genetic basis for differential activities of fluconazole and voriconazole against Candida krusei. Antimicrob. Agents Chemother. 47, 1213-1219. https://doi.org/10.1128/aac.47.4.1213-1219.2003
Gácser, A., Trofa, D., Schäfer, W., Nosanchuk, J.D., 2007. Targeted gene deletion in Candida parapsilosis demonstrates the role of secreted lipase in virulence. J. Clin. Invest. 117, 3049-3058. https://doi.org/10.1172/JCI32294
Garvie, E.I., 1980. Bacterial lactate dehydrogenases. Microbiol. Rev. 44, 106-139. https://doi.org/10.1128/mr.44.1.106-139.1980
Gay, R.J., McComb, R.B., Bowers, G.N., 1968. Optimum Reaction Conditions for Human Lactate Dehydrogenase Isoenzymes as They Affect Total Lactate Dehydrogenase Activity. Clin. Chem. 14, 740-753. https://doi.org/10.1093/clinchem/14.8.740
Gillum, A.M., Tsay, E.Y., Kirsch, D.R., 1984. Isolation of the Candida albicans gene for orotidine-5'-phosphate decarboxylase by complementation of S. cerevisiae ura3 and E. coli pyrF mutations. Mol. Gen. Genet. MGG 198, 179-182. https://doi.org/10.1007BF00328721 Gleason, F.H., Nolan, R.A., Wilson, A.C., Emerson, R., 1966. D(-)-Lactate Dehydrogenase in Lower Fungi. Science 152, 1272-1273. https://doi.org/10.1126/science.152.3726.1272 Graf, K., Last, A., Gratz, R., Allert, S., Linde, S., Westermann, M., Gröger, M., Mosig, A.S., Gresnigt, M.S., Hube, B., 2019. Keeping Candida commensal: how lactobacilli antagonize pathogenicity of Candida albicans in an in vitro gut model. Dis. Model. Mech. 12, dmm039719. https://doi.org/10.1242/dmm.039719
Hall, M.P., Unch, J., Binkowski, B.F., Valley, M.P., Butler, B.L., Wood, M.G., Otto, P., Zimmerman, K., Vidugiris, G., Machleidt, T., Robers, M.B., Benink, H.A., Eggers, C.T., Slater, M.R., Meisenheimer, P.L., Klaubert, D.H., Fan, F., Encell, L.P., Wood, K.V., 2012. Engineered Luciferase Reporter from a Deep Sea Shrimp Utilizing a Novel Imidazopyrazinone Substrate. ACS Chem. Biol. 7, 1848-1857. https://doi.org/10.1021/cb3002478
Hausauer, D.L., Gerami-Nejad, M., Kistler-Anderson, C., Gale, C.A., 2005. Hyphal Guidance and Invasive Growth in Candida albicans Require the Ras-Like GTPase Rsr1p and Its GTPase-Activating Protein Bud2p. Eukaryot. Cell 4, 1273-1286. https://doi.org/10.1128/EC.4.7.1273-1286.2005
Homann, O.R., Dea, J., Noble, S.M., Johnson, A.D., 2009. A Phenotypic Profile of the Candida albicans Regulatory Network. PLOS Genet. 5, e1000783. https://doi.org/10.1371/journal.pgen.1000783
Last, A., Maurer, M., S Mosig, A., S Gresnigt, M., Hube, B., 2021. In vitro infection models to study fungal-host interactions. FEMS Microbiol. Rev. 45, fuab005. https://doi.org/10.1093/femsre/fuab005
Li, D., She, X., Calderone, R., n.d. The Antifungal pipeline: the need is established, are there new compounds? FEMS Yeast Res. https://doi.org/10.1093/femsyr/foaa023
Lindén, S.K., Driessen, K.M., McGuckin, M.A., 2007. Improved In vitro Model Systems for Gastrointestinal Infection by Choice of Cell Line, pH, Microaerobic Conditions, and Optimization of Culture Conditions. Helicobacter 12, 341-353. https://doi.org/10.1111/j.1523-5378.2007.00509.x
Lo, H.J., Köhler, J.R., DiDomenico, B., Loebenberg, D., Cacciapuoti, A., Fink, G.R., 1997. Nonfilamentous C. albicans mutants are avirulent. Cell 90, 939-949. https://doi.org/10.1016/s0092-8674(00)80358-x
Lockwood, L.B., W., G.E., 1936. The Physiology of Rhizopus Oryzae. J. Agric. Res. 53.
Ma, H., Tu, L.-C., Naseri, A., Huisman, M., Zhang, S., Grunwald, D., Pederson, T., 2016. Multiplexed labeling of genomic loci with dCas9 and engineered sgRNAs using CRISPRainbow. Nat. Biotechnol. 34, 528-530. https://doi.org/10.1038/nbt.3526
Makler, M.T., Piper, R.C., Milhous, W.K., 1998. Lactate Dehydrogenase and the Diagnosis of Malaria. Parasitol. Today 14, 376-377. https://doi.org/10.1016/S0169-4758(98)01284-8
Martin, R., Moran, G.P., Jacobsen, I.D., Heyken, A., Domey, J., Sullivan, D.J., Kurzai, O., Hube, B., 2011. The Candida albicans-Specific Gene EED1 Encodes a Key Regulator of Hyphal Extension. PLOS ONE 6, e18394. https://doi.org/10.1371/journal.pone.0018394
Matta, H., Gopalakrishnan, R., Choi, S., Prakash, R., Natarajan, V., Prins, R., Gong, S., Chitnis, S.D., Kahn, M., Han, X., Chaudhary, V., Soni, A., Sernas, J., Khan, P., Wang, D., Chaudhary, P.M., 2018. Development and characterization of a novel luciferase based cytotoxicity assay. Sci. Rep. 8, 199. https://doi.org/10.1038/s41598-017-18606-1
Moyes, D.L., Wilson, D., Richardson, J.P., Mogavero, S., Tang, S.X., Wernecke, J., Höfs, S., Gratacap, R.L., Robbins, J., Runglall, M., Murciano, C., Blagojevic, M., Thavaraj, S., Förster, T.M., Hebecker, B., Kasper, L., Vizcay, G., Iancu, S.I., Kichik, N., Häder, A., Kurzai, O., Luo, T., Krüger, T., Kniemeyer, O., Cota, E., Bader, O., Wheeler, R.T., Gutsmann, T., Hube, B., Naglik, J.R., 2016. Candidalysin is a fungal peptide toxin critical for mucosal infection. Nature 532, 64-68. https://doi.org/10.1038/nature17625
Naglik, J.R., Gaffen, S.L., Hube, B., 2019. Candidalysin: discovery and function in Candida albicans infections. Curr. Opin. Microbiol., Host Microbe Interactions: Fungi • Host-Microbe Interactions: Parasitology 52, 100-109. https://doi.org/10.1016/j.mib.2019.06.002
Niles, A.L., Moravec, R.A., Riss, T.L., 2008. Update on in vitro cytotoxicity assays for drug development. Expert Opin. Drug Discov. 3, 655-669. https://doi.org/10.1517/17460441.3.6.655
Noble, S.M., French, S., Kohn, L.A., Chen, V., Johnson, A.D., 2010. Systematic screens of a Candida albicans homozygous deletion library decouple morphogenetic switching and pathogenicity. Nat. Genet. 42, 590-598. https://doi.org/10.1038/ng.605
Pfaller, M.A., Diekema, D.J., Turnidge, J.D., Castanheira, M., Jones, R.N., 2019. Twenty Years of the SENTRY Antifungal Surveillance Program: Results for Candida Species From 1997-2016. Open Forum Infect. Dis. 6, S79-S94. https://doi.org/10.1093/ofid/ofy358
Riggle, P.J., Andrutis, K.A., Chen, X., Tzipori, S.R., Kumamoto, C.A., 1999. Invasive Lesions Containing Filamentous Forms Produced by a Candida albicans Mutant That Is Defective in Filamentous Growth in Culture. Infect. Immun. 67, 3649-3652. https://doi.org/10.1128/iai.67.7.3649-3652.1999
Riss, T., Niles, A., Moravec, R., Karassina, N., Vidugiriene, J., 2019. Cytotoxicity Assays: In Vitro Methods to Measure Dead Cells, in: Assay Guidance Manual [Internet]. Eli Lilly & Company and the National Center for Advancing Translational Sciences.
Rodrigues, M.L., Nosanchuk, J.D., 2020. Fungal diseases as neglected pathogens: A wake-up call to public health officials. PLoS Negl. Trop. Dis. 14, e0007964. https://doi.org/10.1371/journal.pntd.0007964
Roemer, T., Krysan, D.J., 2014. Antifungal Drug Development: Challenges, Unmet Clinical Needs, and New Approaches. Cold Spring Harb. Perspect. Med. 4, a019703. https://doi.org/10.1101/cshperspect.a019703
Sanchez, A.A., Johnston, D.A., Myers, C., Edwards, J.E., Mitchell, A.P., Filler, S.G., 2004. Relationship between Candida albicans Virulence during Experimental Hematogenously Disseminated Infection and Endothelial Cell Damage In Vitro. Infect. Immun. 72, 598-601. https://doi.org/10.1128/IAI.72.1.598-601.2004
Soliman, S.S.M., Baldin, C., Gu, Y., Singh, S., Gebremariam, T., Swidergall, M., Alqarihi, A., Youssef, E.G., Alkhazraji, S., Pikoulas, A., Perske, C., Venkataramani, V., Rich, A., Bruno, V.M., Hotopp, J.D., Mantis, N.J., Edwards, J.E., Filler, S.G., Chamilos, G., Vitetta, E.S., Ibrahim, A.S., 2021. Mucoricin is a ricin-like toxin that is critical for the pathogenesis of mucormycosis. Nat. Microbiol. 6, 313-326. https://doi.org/10.1038/s41564-020-00837-0 Van den Bossche, S., Vandeplassche, E., Ostyn, L., Coenye, T., Crabb6, A., 2020. Bacterial Interference With Lactate Dehydrogenase Assay Leads to an Underestimation of Cytotoxicity. Front. Cell. Infect. Microbiol. 10, 494. https://doi.org/10.3389/fcimb.2020.00494
Vincken, R., Ruiz-Saenz, A., 2023. A co-culture model system to quantify antibody-dependent cellular cytotoxicity in human breast cancer cells using an engineered natural killer cell line. STAR Protoc. 4, 102224. https://doi.org/10.1016/j.xpro.2023.102224
Wartenberg, A., Linde, J., Martin, R., Schreiner, M., Horn, F., Jacobsen, I.D., Jenull, S., Wolf, T., Kuchler, K., Guthke, R., Kurzai, O., Forche, A., d'Enfert, C., Brunke, S., Hube, B., 2014. Microevolution of Candida albicans in Macrophages Restores Filamentation in a Nonfilamentous Mutant. PLOS Genet. 10, e1004824. https://doi.org/10.1371/journal.pgen.1004824
Weber, K., Bartsch, U., Stocking, C., Fehse, B., 2008. A Multicolor Panel of Novel Lentiviral "Gene Ontology" (LeGO) Vectors for Functional Gene Analysis. Mol. Ther. 16, 698-706. https://doi.org/10.1038/mt.2008.6
Yamamura, M., Boler, J., Valdimarsson, H., 1976. A chromium release assay for phagocytic killing of Candida albicans. J. Immunol. Methods 13, 227-233. https://doi.org/10.1016/0022-1759(76)90069-7
Demaison C, Parsley K, Brouns G, et al. High-level transduction and gene expression in hematopoietic repopulating cells using a human immunodeficiency correction of imunodeficiency virus type 1-based lentiviral vector containing an internal spleen focus forming virus promoter. Hum Gene Ther. 2002;13(7):803-813. doi:10.1089/10430340252898984.

## Claims

1. An *in vitro* method for detecting and/or determining an agent causing cellular damage in a eukaryotic, in particular, mammalian cell, comprising the steps of
a) providing a eukaryotic, in particular, mammalian cell that is genetically modified to stably express an intracellular luciferase enzyme,
b) contacting the cell of a) with at least one agent suspected to cause cellular damage,
c) suitably culturing the cell of b) in a culture medium, and
d) detecting luciferase activity in the culture medium,
wherein a change of the luciferase activity as detected in the culture medium when compared to a genetically modified mammalian cell that was not contacted with the at least one agent is indicative for an agent causing cellular damage in the mammalian cell.

2. An *in vitro* method for detecting and/or determining a cytoprotective agent for a eukaryotic, in particular mammalian cell, comprising the steps of
a) providing a eukaryotic, in particular, mammalian cell that is genetically modified to stably express an intracellular luciferase enzyme,
b) contacting the cell of a) with at least one first agent causing cellular damage,
b) suitably culturing the cell of a) in a culture medium,
c) contacting the agent to be tested with the cell as cultured in b), and optionally suitably culturing the cells in a culture medium
d) detecting luciferase activity in the culture medium, and
wherein a change of the luciferase activity as detected in the culture medium when compared to a genetically modified mammalian cell cultured without the agent to be tested is indicative for cytoprotection in the mammalian cell caused by the agent.

3. The method according to claim 1 or 2, wherein an increase of the luciferase activity as detected in the culture medium is indicative for an agent causing cellular damage, and wherein a decrease of the luciferase activity as detected in the culture medium is indicative for an agent causing cytoprotection.

4. The method according to any one of claims 1 to 3, further comprising the step of concluding on the pharmacological safety of the agent based on the detecting.

5. The method according to any one of claims 1 to 4, wherein the agent causing cellular damage or cytoprotection is selected from agents related to senescence, infection, environmental factors, cytotoxicity, cancer, radiation or physical damage, a pharmaceutically active compound, a drug, in particular a small molecule drug, an antibiotic, an antibody, a peptide, and an immune cell.

6. The method according to claim 5, wherein the cell is infected with an infectious agent, such as, for example a bacterium, a fungus, or a virus, such as, for example, *Candida, Yersinia,* Zika, HIV, Coronavirus, RSV, or West Nile Virus.

7. The method according to any one of claims 1 to 6 wherein the eukaryotic cell is selected from the group consisting of insect cells, fungal cells, e.g., yeast, and mammalian cell selected from the group consisting of epithelial cells, vaginal cells, intestinal cells, lung cells, immortalized cells, cancer cells, and cell lines thereof.

8. The method according to any one of claims 1 to 7, wherein genetically modifying the cell comprises transfection of the cell with a recombinant lentiviral vector encoding the luciferase.

9. The method according to any one of claims 1 to 8, wherein the luciferase is a genetically modified luciferase, such as Nano-Luc (Nluc).

10. The method according to any one of claims 1 to 9, wherein detecting luciferase activity in the culture medium comprises centrifuging down of the cell debris, collecting a suitable amount of supernatant, diluting the supernatant with a suitable buffer, adding luciferase substrate, and measuring the luminescence as generated.

11. The method according to any one of claims 1 to 10, further comprising at least one additional step of determining the number of cells in the culture medium based on determining the luciferase activity of the genetically modified cells as cultured, preferably in a lysate.

12. The method according to any one of claims 1 to 11, which is, at least in part, automated.

13. A diagnostic kit comprising materials for performing the method according to any one of claims 1 to 12, in particular a luciferase substrate, a genetically modified mammalian cell line stably expressing Nluc, buffers, triton, positive controls, culture medium, optionally an agent to be tested, and/or instructions for use.

14. Use of the diagnostic kit according to claim 13 for detecting and/or determining cellular damage in a mammalian cell, for detecting and/or determining a cytoprotective agent in a damaged cell, for screening for an agent causing cytoprotection in a cell, or for determining the pharmacological safety of an agent according to any one of claims 1 to 12.
